# EUROPÄISCHE PATENTSCHRIFT

(11) **EP 0 367 133 B1**
(45) Veröffentlichungstag und Bekanntmachung des Hinweises auf die Patenterteilung: **04.08.1993**
(21) Anmeldenummer: 89119968.9
(22) Anmeldetag: 27.10.1989
(51) Int. Cl.: G01N 33/28

(54) **Vorrichtung zur Prüfung von Kraftstoff - und Öladditiven**
Apparatus for examining food and oil additives
Dispositif pour l examen des additifs dans de l huile et dans des carburants

(30) Priorität: 04.11.1988 DE 3837470
(43) Veröffentlichungstag der Anmeldung: 09.05.1990
(73) Patentinhaber: BASF Aktiengesellschaft, 67063 Ludwigshafen (DE)
(72) Erfinder: Fikentscher, Rolf, Dr., D-6700 Ludwigshafen (DE); Oppenlaender, Knut, Dr., D-6700 Ludwigshafen (DE); Schwen, Roland, Dr., D-6701 Friedelsheim (DE)

(56) Entgegenhaltungen:
- DE-C- 888 622
- GB-A- 968 645
- US-A- 3 059 467
- US-A- 3 200 638

## Beschreibung

Die Erfindung betrifft eine Vorrichtung zur Prüfung von Kraftstoff- und Öladditiven für Verbrennungsmotoren, bestehend aus einem geneigt angeordneten, rinnenförmigen Prüfkörper aus Metall mit einer Heizeinrichtung zum Temperieren der Kraftstoffgemischprobe.

Bei Ottomotoren besteht die Gefahr der Verschmutzung [M. Völtz, Erdöl und Kohle-Erdgas-Petrochemie, 40, 270 (1987)], insbesondere des Einlaßsystems [Automotive Engineering 96, 68 (April 1988)] und dabei des Einlaßventiltellers [Automotive Enineering 95, 69 (November 1987)] in Form teerähnlicher Ablagerungen und Kohle.

Entsprechende Schäden werden vom Markt gemeldet und sind in Motoren-Prüfstandsversuchen und in Fahrversuchs-Flotten prinzipell nachvollziehbar. Dabei bestehen aber folgende Nachteile:
- Die Motorenversuche und in noch höherem Maße die Flottenteste sind teuer und zeitaufwendig.
- Reproduzierbarkeit ist nur mit großem Aufwand gegeben bzw. möglich, weil
- der Einfluß, z.B. der Kraftstoffsorte, d.h. der individuellen Kraftstoff-Partie, unberücksichtigt bleiben muß und
- der gegenseitige Einfluß von Kraftstoff einschließlich deren Additive und Schmieröl eine Vielzahl von Testkombinationen verlangt.

Aus der Patentschrift US-A-3 059 467 ist ein Labortestgerät zum Prüfen von Kraftstoffgemischen für Verbrennungsmotoren bekannt, welches aus einer geneigt angeordneten Prüfrinne besteht. Die Prüfrinne ist mittels eines umgebenden, an eine Heizeinrichtung angeschlossenen Gehäuses auf eine vorgewählte Temperatur indirekt heizbar. Diese indirekte Heizung ermöglicht jedoch keinen bestimmten reproduzierbaren Temperaturverlauf entlang der Rinne. Praxisnahe Tests erfordern bestimmte Temperaturprofile. Ferner stellt das Gehäuse eine Gefahr hinsichtlich der darin entstehenden Kraftstoffdämpfe dar.

Es bestand daher die Aufgabe, eine Vorrichtung zur Prüfung von Kraftstoff- und Öladditiven zu entwickeln, mit der die Kraftstoffgemischprobe einem gewünschten, ansteigenden Temperaturverlauf ausgesetzt werden kann.

Die Aufgabe wurde durch eine Vorrichtung der eingangs geschilderten Art gelöst, bei der dem Prüfkörperende mit der niederen Anfangstemperatur eine Dosiereinrichtung mit einer Zuführung für die Gemischprobe zugeordnet ist und die Heizeinrichtung in Form einer elektrischen Widerstandheizung durch den mit seinen beiden Enden an eine Spannungsquelle angeschlossenen Prüfkörper selbst gebildet ist, wobei zur Erzeugung des ansteigenden Temperaturverlaufs sich die Querschnittsfläche des Körpers vom Zuführungsende aus kontinuierlich verringert.

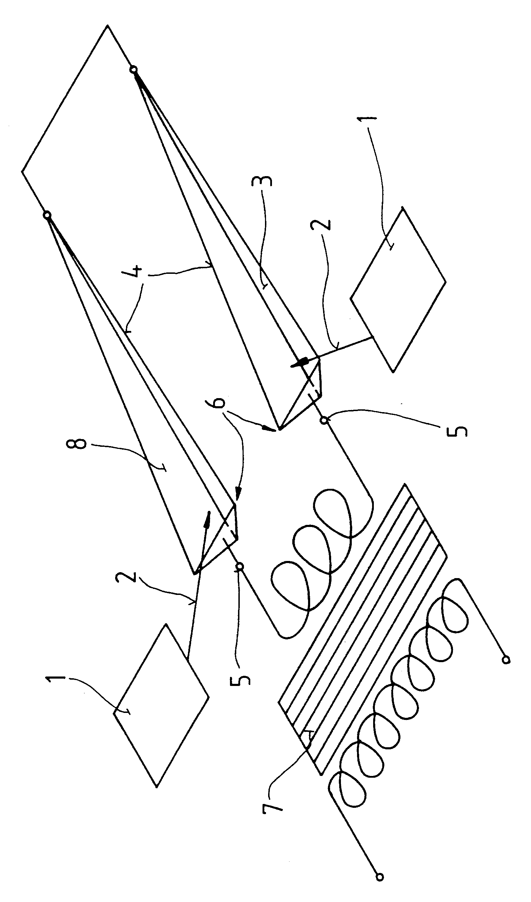

Die Erfindung ist anhand eines in der Zeichnung schematisch dargestellten Ausführungsbeispiels nachfolgend näher beschrieben.

Eine Probemenge des zu prüfenden Kraftstoff-/Öl-/Additiv-Gemisches wird mittels einer Dosierpumpe 1 über eine Zuführung 2 auf einen mit etwa 10 Winkelgraden nach unten geneigt angeordneten, rinnenförmigen Prüfkörper 3 aus Metall gegeben. Auf dem Prüfkörper fließt die Gemischprobe langsam zum anderen Ende und soll dabei einer steigenden Temperatur ausgesetzt sein, die in einem dem Motorbetrieb entsprechenden Bereich liegt.

Hierzu ist der Prüfkörper mit einer Heizeinrichtung 4 ausgestattet, welche den Körper auf einem Temperaturverlauf hält, der vom Zuführungsende aus ansteigt, beispielsweise von 200°C bis auf 400°C. Die Heizeinrichtung besteht aus einer elektrischen Beheizung, bei der der Prüfkörper 3 mit seinen beiden Enden an eine Spannungsquelle 5 angeschlossen ist und sich die Querschnittsfläche des Körpers vom Zuführungsende 6 aus für ein Ansteigen des elektrischen Widerstandes und damit zur Erzeugung eines ansteigenden Temperaturverlaufs kontinuierlich verringert. Die Querschnittsänderung kann durch abnehmende Materialstärke oder sich verringernde Körperbreite oder aber durch beides erhalten werden. Da leichtentzündliche Stoffe erhitzt werden, empfiehlt sich ein Niederspannungsanschluß mittels eines geeigneten Transformators 7.

Es ist zweckmäßig, eine von der Beschaffenheit des Prüfkörpers abhängige Vorheizzeit vorzusehen, beispielsweise eine Stunde, um ein stabiles Temperaturprofil sicherzustellen. Die Messung der Oberflächentemperaturen kann z.B. mit Hilfe von Thermoelementen oder Oberflächenthermometern oder Thermocolorfarben erfolgen.

Zur Durchführung mehrerer Messungen gleichzeitig können mehrere Prüfkörper zusammengeschaltet werden, wobei gleiches Material, gleiche Materialstärke und gleiche Umrißformen ein einheitliches Temperaturprofil garantieren, wenn die Prüfkörper hintereinander geschaltet werden.

Für die Dosierung des Gemisches wird davon ausgegangen, daß ein typischer Viertakt-Ottomotor, der zur Einlaßventilverschmutzung neigt, einen Ölverlust pro Ventilführung von 1 g/h hat und 2 Liter Krafstoff/Stunde und Zylinder verbraucht. Wollte man diese Kraftstoffmenge auf einem geheizten Prüfkörper verdampfen, so würde der Körper davon im Anfangsbereich zu stark gekühlt werden und die Explosionsgefahr zu groß sein. Daher werden beispielsweise 90 % der 2 Liter Kraftstoff über eine Laborkolonne abdestilliert und der Rückstand von 10 % für die Messung verwendet. Dazu wird die kritische Ölmenge von der zu prüfenden Qualität, d.h. 1 g, und das zu prüfende Kraftstoffadditiv, z.B. 1000 ppm, gegeben. Die Zugabe des Gemisches auf den Prüfkörper erfolgt dann innerhalb einer Stunde kontinuierlich.

Unter der Wärmeeinwirkung auf das Gemisch bilden sich nach einer gewissen Fließstrecke auf dem Prüfkörper feste Rückstandsprodukte aus teerähnlichen Produkten und Kohle. Deren Gewicht und die Fließstrecke werden zur Bewertung der Additive festgehalten:
Eine lange Fließstrecke und/oder niedriges Rückstandsgewicht weisen auf ein wirksames Additiv hin.

Im Motorenbetrieb gelangen beim Kaltstart flüssige Kraftstoffanteile auf das Einlaßventil. Um deren Waschwirkung zu simulieren, werden anschließend die Prüfkörper mit den Rückständen, z.B. 2 Minuten lang in ca. 50°C warmem Benzin gewaschen und die verbleibenden Rückstände nochmals gewogen. Niedrige Werte zeigen auch hier ein gutes Resultat an.

Eine besonders vorteilhafte Prüfanordnung besteht darin, daß ein zweiter, gleicher Prüfkörper 8 für eine parallel durchzuführende Bezugsmessung eines Kraftstoff-Öl-Gemisches ohne Additive vorgesehen ist. Die beiden Prüfkörper 3 und 8 sind in Reihe an die Spannungsquelle 5 angeschlossen, um übereinstimmende Temperaturprofile zu erhalten. Der Prüfvorgang läuft wie vorstehend beschrieben ab, wobei die Wirksamkeit des zu prüfenden Additivs auf einfache Weise durch die Differenz der Rückstandsgewichte und der Fließstrecken der beiden Messungen zu bestimmen ist.

Zur Veranschaulichung der erfindungsgemäßen Additivprüfung ist nachfolgend eine durchgeführte Messung dargestellt.

### Beispiel

- Kraftstoff:: Superkraftstoff
- Öl:: Mehrbereichsmotorenöl RL 51

Additiv 1000 ppm bzw. ohne Additiv

Der Prüfkörper besteht aus Stahl St 37 und ist ohne die Stromanschlüsse 46 cm lang; seine Querschnittsfläche beginnt mit 55 mm² und endet mit 35 mm². Nach der Aufheizperiode beträgt die Spannung für die Prüfkörperheizung 1,4 V und die Stromaufnahme 260 A. Die Prüfkörpertemperatur liegt zwischen 200°C am Zuführungsende und 450°C am Auslaufende. Der Prüfkörper ist um 10° gegenüber der Waagrechten geneigt.

### Prüfungsergebnis:

| | Fließstrecke [cm] | Gewicht des Rückstands [g] vor dem Waschen nach dem Waschen | |
|---|---|---|---|
| Bezugsmessung | 23 | 0,31 | 0,23 |
| Messung mit Additiv A | 30,5 | 0,24 | 0,14 |
| Messung mit Additiv B | 25 | 0,57 | 0,40 |

Daraus folgt: Additiv A und Additiv B sind wirksam, weil sie längere Laufstrecken als die Bezugsmengen aufweisen, und Additiv A ist besser als Additiv B, weil es sowohl die längere Laufstrecke als auch die kleinste Rückstandsmenge sowohl vor als auch nach dem Waschen hat.

## Patentansprüche

1. Vorrichtung zur Prüfung von Kraftstoff- und Öladditiven für Verbrennungsmotoren, bestehend aus einem geneigt angeordneten, rinnenförmigen Prüfkörper (3) aus Metall mit einer Heizeinrichtung (4) zum Temperieren der Kraftstoffgemischprobe, dadurch gekennzeichnet, daß dem Prüfkörperende (6) mit der niederen Anfangstemperatur eine Dosiereinrichtung (1) mit einer Zuführung (2) für die Gemischprobe zugeordnet ist und die Heizeinrichtung in Form einer elektrischen Widerstandheizung durch den mit seinen beiden Enden an eine Spannungsquelle (5) angeschlossenen Prüfkörper (3) selbst gebildet ist, wobei zur Erzeugung des ansteigenden Temperaturverlaufs sich die Querschnittsfläche des Körpers vom Zuführungsende (6) aus kontinuierlich verringert.

## Claims

1. An apparatus for testing fuel additives and oil additives for internal combustion engines, comprising an inclined, channel-like metal test element (3) having a heating means (4) for heating the fuel mixture sample, wherein a metering means (1) having a feed (2) for the sample of mixture is coordinated with that end (6) of the test element which is at the lower initial temperature and wherein the heating means in the form of an electrical resistance heater is formed by the test element (3) itself, whose two ends are connected to a voltage source (5), the cross-sectional area of the element decreasing continuously starting from the feed end (6) in order to produce the increasing temperature profile.

## Revendications

1. Dispositif de contrôle d'additifs pour carburant et huile pour moteurs à combustion interne, composé d'une sonde en métal (3) en forme de goulotte, disposée inclinée, avec un moyen de chauffage (4) pour réchauffer l'échantillon de mélange de carburant, caractérisé en ce qu'un moyen de dosage (1), avec une amenée (2) pour l'échantillon de mélange, est associé à l'extrémité de sonde (6) dont la température initiale est faible et le moyen de chauffage est réalisé sous forme d'un chauffage à résistance électrique dans la sonde (3) même, raccordée à une source de tension (5) par ses deux extrémités, la surface de la section transversale de la sonde allant en diminuant de façon continue, depuis l'extrémité d'amenée (6), en vue de produire une évolution croissante de la température.
